# EUROPÄISCHE PATENTSCHRIFT

(11) **EP 1 214 278 B1**
(45) Veröffentlichungstag und Bekanntmachung des Hinweises auf die Patenterteilung: **26.05.2004**
(21) Anmeldenummer: 00964049.1
(22) Anmeldetag: 29.08.2000
(51) Int. Cl.: C07B 39/00, C07C 17/20, C07C 41/22, C07C 51/58, C01B 17/45

(54) **REGENERIERUNG VON ONIUMFLUORID-HF-ADDUKTEN**
REGENERATION OF ONIUM FLUORIDE-HF ADDUCTS
REGENERATION D'ADDITIFS DE FLUORURE D'ONIUM-HF

(30) Priorität: 04.09.1999 DE 19942373; 24.11.1999 DE 19956365
(43) Veröffentlichungstag der Anmeldung: 19.06.2002
(73) Patentinhaber: Solvay Fluor und Derivate GmbH, 30173 Hannover (DE)
(72) Erfinder: BRAUN, Max, 30900 Wedemark (DE); PALSHERM, Stefan, 30890 Barsinghausen (DE)
(74) Vertreter: Fischer, Reiner
(86) Internationale Anmeldenummer: PCT/EP2000/008396
(87) Internationale Veröffentlichungsnummer: WO 2001/017931

(56) Entgegenhaltungen:
- EP-A- 0 005 810
- DE-A- 19 942 374
- US-A- 4 372 938
- US-A- 5 847 245

## Beschreibung

Die Erfindung bezieht sich auf ein Verfahren zur Regenerierung von verbrauchten Ammoniumfluorid-HF-Addukten.

Addukte von Ammoniumfluoriden und HF können als Fluorierungsreagenz eingesetzt werden. Die EP-A-0 901 999 beispielsweise offenbart die Herstellung von Sevoflurane aus dem entsprechenden, fluorierten Ether mittels HF und Amin. Die Aufarbeitung erfolgt unter Wasserzusatz. Damit wird das Oniumfluorid-HF-Addukt zerstört.

Die US-A 4,372,938 offenbart die Herstellung von SF₄ mittels Ammoniumfluorid-Addukten. Die verbrauchten Oniumfluorid-HF-Addukte werden durch Kontaktieren mit Fluorwasserstoff regeneriert und erneut für die SF₄-Herstellung eingesetzt. Bei den verwendeten Aminen handelt es sich um stickstoffhaltige, heterocyclische aromatische Verbindungen.

Die europäische Patentanmeldung EP-A-0 005 810 offenbart die Verwendbarkeit von HF-Addukten von Aminen als Fluorierungsmittel. Eine bevorzugte Verfahrensvariante sieht vor, den bei der Reaktion freiwerdenden Fluorwasserstoff zu binden, indem man in Gegenwart eines Lösungsmittels ausreichender Basizität arbeitet; bevorzugt sind Säureamide als Lösungsmittel. Die erhaltenen Säureamid-Fluorwasserstoff-Gemische können aufgearbeitet werden und, nach Titration mit Lauge und Aminzugabe, wieder verwendet werden.

Das US-Patent US-A-4 372938 offenbart die Herstellung von Schwefeltetrafluorid aus Schwefelchloriden unter Verwendung von Addukten von Fluorwasserstoff und stickstoffhaltigen heterocyclischen aromatischen Aminen. Bei der Umsetzung gebildete Hydrochloride des aromatischen Amins können mit Fluorwasserstoff regeneriert werden.

Aufgabe der vorliegenden Erfindung ist es, ein verbessertes Regenerierungsverfahren anzugeben. Diese Aufgabe wird durch das Verfahren der vorliegenden Erfindung gelöst.

Das erfindungsgemäße Verfahren zur Herstellung von Fluor enthaltenden Verbindungen aus Chlor oder Brom enthaltenden Verbindungen durch Chlor-Fluor-Austausch oder Brom-Fluor-Austausch unter Verwendung von Oniumfluorid-HF-Addukten als Reagenz oder Katalysator sieht vor, daß man verbrauchtes Oniumfluorid-HF-Addukt kontinuierlich oder batchweise mittels HF in Anwesenheit einer flüssigen Carbonsäure regeneriert und/oder daß man verbrauchte Trialkylammoniumfluorid-HF-Addukte oder verbrauchte HF-Addukte cyclischer, gesättigter Amine mittels HF regeneriert.

"Verbraucht" bedeutet, daß im Addukt das Verhältnis A-min:HF unerwünscht groß geworden ist und/oder der Gehalt an HCl oder HBr unerwünscht hoch geworden ist. Bei der Regenerierung wird insbesondere der Gehalt an HCl (bzw. HBr) auf ein akzeptables Maß reduziert, z. B. auf weniger als 1 Mol HCl pro Mol Amin oder NH₃. Dabei kann der HF-Gehalt auf die gewünschte Menge gebracht werden (durch Zusatz oder Abdampfen oder Beimischen von Addukten mit geeigneter Konzentration).

Die Regenerierung wird zweckmäßig im Autoklaven oder Druckbehälter unter HF-Zusatz bei erhöhter Temperatur, z. B. im Bereich von 80 bis 120 °C, durchgeführt. Dabei wird HCl bzw. HBr freigesetzt und kann nach Öffnung des Autoklaven aus der Gasphase entfernt werden. Beispielsweise kann man Inertgas durch den Autoklaven leiten und die Gasphase entfernen.

Es hat sich gezeigt, daß es vorteilhaft ist, wenn bei der Fluorierungsreaktion nicht im wesentlichen das gesamte HF für die Fluorierung verbraucht wird. Zwar kann selbst dann eine Regenerierung durchgeführt werden, wenn das Addukt im wesentlichen vollständig zu Ammoniumchlorid oder Oniumchlorid bzw. zum entsprechenden Bromid umgewandelt ist. Die Regenerierung ist aber leichter durchzuführen, wenn das Verhältnis von Amin zu HF bei der Fluorierungsreaktion nicht unter 1 sinkt.

Selbstverständlich kann man das Verhältnis von Amin zu Fluorwasserstoff im regenerierten Produkt einstellen. Für manche Anwendungszwecke ist es wünschenswert, daß das Verhältnis von Amin zu Fluorwasserstoff im Addukt bei einem Wert zwischen 1:1,1 und 1:3,5, vorzugsweise zwischen 1:2 und 1:3 liegt. Dies kann man beispielweise dadurch erzielen, daß man überschüssigen Fluorwasserstoff im regenerierten Produkt durch Erhitzen oder Destillation abtrennt.

Gemäß der US-A 4,472,938 werden bei der Herstellung von Schwefeltetrafluorid bevorzugt Ammoniumfluorid-HF-Addukte eingesetzt, die auf stickstoffhaltigen aromatischen Verbindungen basieren, z. B. auf Pyridiniumfluorid-HF-Addukten. Beim Regenerierungsverfahren ohne Säurezusatz der vorliegenden Erfindung setzt man gemäß einer Ausführungsform verbrauchte Trialkylammoniumfluorid-HF-Addukte oder HF-Addukte cyclischer, gesättigter Amine ein. Diese weisen beim Regenerierungsverfahren Vorteile auf. Es wurde nämlich festgestellt, daß verbrauchte Trimethylammoniumfluorid- oder Triethylammoniumfluorid-HF-Addukte, also Addukte von Aminen mit kurzen Alkylketten, HCl bei der Regenerierung leicht freisetzen. Verbrauchte Trialkylammoniumfluorid-HF-Addukte mit Alkylgruppen, die mindestens drei C-Atome aufweisen, neigen weniger dazu, Feststoffe zu bilden. Dies ist bei der Regenerierung ohne Säurezusatz natürlich von Vorteil. Auch verbrauchte HF-Addukte von Aminen, in denen der Stickstoff in ein gesättigtes 5- oder 6-Ringsystem eingebaut ist, das auch noch Heteroatome wie Sauerstoff enthält, können so regeneriert werden, z. B. Addukte von Piperidin, Pyrrolidin.

Ohne Säurezusatz regeneriert man besonders bevorzugt verbrauchte Trimethylamin-HF-Addukte, Triethylamin-HF-Addukte, Tripropylamin-HF-Addukte und Tributylamin-HF-Addukte.

Gemäß einer Variante des erfindungsgemäßen Verfahrens regeneriert man die verbrauchten Addukte unter Zusatz einer flüssigen Carbonsäure (dieser Begriff umfaßt auch Dicarbonsäuren oder Tricarbonsäuren). Bevorzugt sind durch Halogenatome, insbesondere Fluoratome, substituierte Carbonsäuren, insbesondere mit insgesamt 2 bis 4 Kohlenstoffatomen. Trifluoressigsäure als Zusatzmittel ist besonders bevorzugt. Addukte mit kurzkettigen Alkylgruppen neigen zur Feststoffbildung; dem kann mittels einer flüssigen Carbonsäure, insbesondere Trifluoressigsäure, entgegengearbeitet werden. Bei verbrauchten Addukten von Aminen mit längerkettigen Alkylgruppen wird das Austreiben von enthaltenem HCl gefördert, wenn man unter Zusatz einer Carbonsäure arbeitet. Oftmals reicht schon ein Zusatz von bis zu 10 Mol.-% der Säure, bezogen auf das als 100 Mol.-% berechnete, verbrauchte Onium-HF-Addukt, aus. Selbstverständlich kann man auch mehr Säure zugeben, beispielsweise bis hin zu 20 Mol.-% oder mehr. 8.0 Mol-% werden als Obergrenze angesehen. Bei mehr als 80 Mol-% zugesetzter Säure kommt es möglicherweise zu-Nebenreaktionen.

Gemäß einer ganz besonders bevorzugten Ausführungsform regeneriert man verbrauchte Trialkylammoniumfluorid-HF-Addukte, unabhängig von der Länge der Alkylgruppen am Stickstoff, unter Zusatz einer flüssigen Carbonsäure, vorzugsweise Trifluoressigsäure. Vorteil ist die verringerte Neigung zur Bildung von Feststoffen und das leichte Austreiben von HCl-Gas.

Das erfindungsgemäße Verfahren unter Säurezusatz kann generell zur Regenerierung von HF-Addukten von Ammoniak und Aminen dienen. Bei diesen Aminen kann es sich um primäre, sekundäre und tertiäre Amine handeln. Die Substituenten sind vorzugsweise lineare oder verzweigte Alkylgruppen mit 1 bis 12 Kohlenstoffatomen. Man kann auch Addukte von zyklischen Aminen wie Pyrrolidin, N-Methylpyrrolidin oder Piperidin einsetzen. Auch Amine mit aromatischen Substituenten wie Anilin oder Amine mit Stickstoff in einem aromatischen Ringsystem wie Pyridin können regeneriert werden. Besonders gut geeignet ist das Regenerierungsverfahren für HF-Addukte der genannten zyklischen bzw. aromatischen Amine sowie für sekundäre und tertiäre Amine, insbesondere wenn diese sekundären und tertiären Amine mit 1 bis 5, vorzugsweise 2 bis 4 Kohlenstoffatomen pro Substituent substituiert sind. Bevorzugte Substituenten sind Methyl, Ethyl, n-Propyl, i-Propyl, n-Butyl, i-Butyl.

Das erfindungsgemäße Verfahren gestattet die Wiederverwendung von verbrauchten HF-Addukten von Ammoniak und Aminen aus Fluorierungsreaktionen.

Mit dem erfindungsgemäßen Regenerierungsverfahren kann man beispielsweise verbrauchte Addukte aus der Herstellung von Säurefluoriden aus Säurechloriden, z. B. der Herstellung von Carbonsäurefluoriden oder Sulfurylfluorid aus den entsprechenden Chloriden, regenerieren. Auch können verbrauchte Addukte, die aus der Herstellung von Fluoralkylgruppen aus Chloralkylgruppen stammen. Beispiele für solche Reaktionen sind die Herstellung von Fluorkohlen(wasser)stoffen, Fluorchlorkohlen(wasser)stoffen und den entsprechenden Bromderivaten aus entsprechenden Chlorverbindungen durch Fluor-Chlor-Austausch oder Fluor-Brom-Austausch. Weiteres Beispiel für solche Reaktionen ist die Herstellung von Fluoralkylethern aus entsprechenden Chlor- oder Brom-Alkylethern.

Eine besondere Ausführungsform betrifft die Regeneration bei mehrstufigen Fluorierungsverfahren. Bei manchen mehrstufigen Fluorierungsverfahren sind die Bedingungen hinsichtlich des erforderlichen Reaktionsmediums unterschiedlich. Dies ist beispielsweise bei der Herstellung von Sulfurylfluorid aus Sulfurylchlorid der Fall. Es wurde festgestellt, daß die zweite Fluorierungsstufe, nämlich die Umwandlung von Sulfurylchlorofluorid in Sulfurylfluorid, nur dann befriedigend abläuft, wenn die Ausgangsverbindung Sulfurylchlorofluorid mit einem Addukt von Oniumfluorid und HF erfolgt, in welchem das Verhältnis zwischen Amin und Fluorwasserstoff nicht oberhalb von 1:3,5 liegt. Die erste Stufe, die Fluorierung von Sulfurylchlorid zu Sulfurylchlorofluorid, ist dagegen vom Gehalt an Fluorwasserstoff im Reaktionsmedium unabhängig. Gemäß einer speziellen Ausführungsform der Erfindung ist vorgesehen, daß man in Fällen, in denen der Fluorwasserstoffgehalt im Reaktionsmedium unwichtig ist; gleichzeitig die Regenerierung des verbrauchten Addukts vornimmt. Im Falle der Herstellung von Sulfurylfluorid geht man dann so vor, daß man in einem ersten Reaktor verbrauchtes Addukt mit einem großen Überschuß HF regeneriert und gleichzeitig das im Reaktor vorhandene Sulfurylchlorid zu Sulfurylchloridfluorid fluoriert. Dabei ist es nicht notwendig, daß das gesamte Sulfurylchlorid umgesetzt wird. Man destilliert dann nach der Regenerierung Fluorwasserstoff ab, so daß das Amin-HF-Verhältnis auf den gewünschten Wert von unterhalb 1:3,5 gebracht wird. Der Reaktorinhalt kann dann entweder weiter umgesetzt werden zu Sulfurylfluorid oder man überführt ihn in einen anderen Reaktor zur Durchführung dieser Reaktion.

Die folgenden Beispiele sollen die Erfindung weiter erläutern, ohne sie in ihrem Umfang einzuschränken. Die Beispiele belegen, daß sich nicht nur HF-Addukte mit wechselndem Gehalt an HCl regenerieren lassen, sondern sogar reines Hydrochlorid. Ein vollständiges Regenerieren in dem Sinne, daß HCl vollkommen eliminiert wird, ist nicht notwendig, aber möglich. Anwendungsbeispiele haben ergeben, daß auch HF-Addukte mit einem Restgehalt von HCl durchaus als Fluorierungskatalysator oder Fluorierungsmittel brauchbar sind.

Regeneriertes Produkt der Formel NH₃ x (1,1-9)HF x (0,001-1)HCl bzw. Amin x (1,1-9)HF x (0,001-1)HCl ist ein Verfahrens-Produkt, bevorzugt mit 1,1 bis 3 HF und 0,001 bis 0,5 HCl. "Amin" steht für NR₃ mit R = C1-C4-Alkylgruppen.

### Beispiel 1:

### Recycling von NBu₃ x 1,7 HCl zu NBu₃ x Y HF unter Einsatz von wenig HF

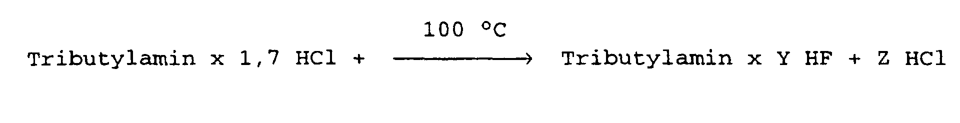

### Ansatz:

| **Stoff** | **Molmasse** | **Masse in g** | **Mol** |
|---|---|---|---|
| Tributylamin x 1,7 HCl | 247,34 | 82,17 | 0,33 |
| HF | 20,01 | 50 | 2,5 |

In einem Laborautoklav wurde Tributylamin x 1,7 HCl vorgelegt. Nach dem verschließen wurde 50 g HF zugegeben und ca. 3 h bei 100 °C Reaktorinnentemperatur gekocht. Der Autoklav wurde dann auf ca. 60 °C Reaktorinnentemperatur abgekühlt und die Gasphase bis zu Atmosphärendruck im Autoklaven in eine Waschflasche mit Wasser geleitet.

Bestimmt nach der Chlorid- und Fluoridanalyse der Waschflasche hatte der im Autoklaven verbliebene Katalysator nun eine Zusammensetzung von Tributylamin x 0,62 HCl x 7,3 HF.

Das Beispiel zeigt, daß selbst vollständig unter Bildung von Hydrochlorid verbrauchtes HF-Addukt regeneriert werden kann. Der regenerierte Katalysator konnte wieder in Fluorierungsreaktionen eingesetzt werden. Das folgende Beispiel zeigt, daß der HCl-Gehalt dieses Produktes noch weiter abgesenkt werden kann.

### Beispiel 2:

### Weitere Verringerung des HCl-Gehaltes in NBu₃ x 0,62 HCl x 7,3 HF zu NBu₃ x Y HF unter Einsatz eines Überschusses an HF

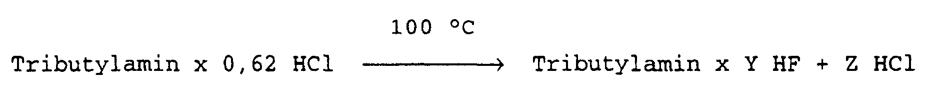

### Ansatz:

| **Stoff** | **Molmasse** | **Masse in g** | **Mol** |
|---|---|---|---|
| Tributylamin x 0,62 HCl x 7,3 HF | 354,3 | 116,83 | 0,33 |
| HF | 20,01 | 107 | 5,35 |

Zur im Autoklaven verbliebenen Mischung von Tributylamin x 0,62 HCl x 7,3 HF wurden erneut nun 107 g HF gegeben und bei ca. 100 °C nun über Nacht gekocht. Der Autoklav wurde dann auf ca. 60 °C Reaktorinnentemperatur abgekühlt und die Gasphase bis zu Atmosphärendruck in eine Waschflasche mit Wasser geleitet. Berechnet anhand der Chlorid- und Fluoridanalyse der Waschflasche hatte der im Autoklaven verbliebene Katalysator nun eine Zusammensetzung von Tributylamin x 0,005 HCl x 4,89 HF. Diese Zusammensetzung wurden durch Direktanalyse des verbliebenen Rückstandes im Autoklaven bestätigt. Die HCl war somit nahezu vollständig ausgetrieben worden. Zersetzungsprodukte des Amins wurden nicht gefunden.

Die erhaltene Zusammensetzung war hervorragend brauchbar als Fluorierungsreagenz und Fluorierungskatalysator.

### Beispiel 3:

### Recycling von NEt₃ x 1,0 HCl zu NEt₃ x Y HF unter Einsatz eines Überschusses an HF

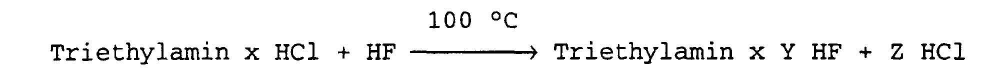

### Ansatz:

| **Stoff** | **Molmasse** | **Masse in g** | **Mol** |
|---|---|---|---|
| Tributylamin x HCl | 137,65 | 37,93 | 0,28 |
| HF | 20,01 | 107,7 | 5,38 |

### Durchführung:

In einem Laborautoklav wurde Triethylamin Hydrochlorid vorgelegt und verschlossen. Danach wurde HF zu gegeben und über Nacht bei 100 °C Reaktorinnentemperatur gekocht. Anschließend wurde bei 100 °C Reaktortemperatur die Gasphase bis zu Atmosphärendruck in eine mit Wasser gefüllte Waschflasche abgelassen. Berechnet anhand der Chlorid- und Fluoridanalyse der Waschflasche hatte der im Autoklaven verbliebene Katalysator nun eine Zusammensetzung von Triethylamin x 0,09 HCl x 5,35 HF. Diese Zusammensetzung wurden durch Direktanalyse des verbliebenen Rückstandes im Autoklaven bestätigt. Die HCl war somit nahezu vollständig ausgetrieben worden. Zersetzungsprodukte des Amins wurden nicht gefunden.

### Beispiel 4:

### Einstellung des Verhältnisses von Amin zu HF auf 2,8 bei der Katalysatormischung aus Versuch 3

### - Entfernung

### Durchführung:

Die in Versuch 3 erhaltene Mischung Triethylamin x 0,09 HCl x 5,35 HF wurde in eine PFA-Flasche mit Fritte gefüllt und mit trockenem Stickstoff die überschüssige HF ausgetrieben. Nachdem das Gewicht der Flasche 30 min konstant geblieben ist, wurde noch 10 min ein Vakuum von 10⁻³ mbar angelegt um wirklich alle Restmengen HF zu entfernen. Der so erhaltene Katalysator hatte nach Chlorid-, Fluorid- und Aminanalyse nun eine Zusammensetzung von Triethylamin x 2,8 HF. HCl wurde nicht mehr gefunden. Die Recycelbarkeit des Katalysators wurde hiermit bewiesen.

Durch die vorgenommene Einstellung des Amin-HF-Verhältnisses werden die nucleophilen Eigenschaften des Adduktes wiederhergestellt. Es eignet sich jetzt hervorragend als Reagenz zur Herstellung von So₂F₂ aus So₂Cl₂.

### Beispiel 5 und 6:

### Variation der Zeitdauer der Regenerierung

### Beispiel 5:

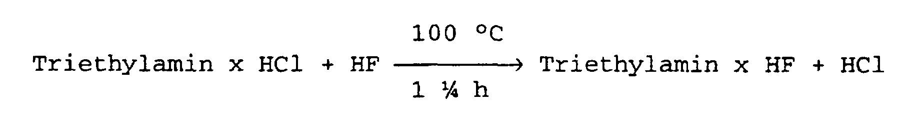

### Ansatz:

| **Stoff** | **Molmasse** | **Masse in g** | **Mol** |
|---|---|---|---|
| Tributylamin x HCl | 137,65 | 10,00 | 0,07 |
| HF | 20,01 | 16,5 | 0,82 |

### Durchführung:

In einem Laborautoklav wurde Triethylamin Hydrochlorid vorgelegt und verschlossen. Danach wurde HF zu gegeben und 1 ¼ bei ca. 100 °C Reaktortemperatur gekocht (Autoklav wurde 15 min vorgeheizt). Nach dieser Zeit wurde die Gasphase innerhalb von 15 min im Ölbad stehend abgelassen und analysiert (Probe 1). Der Reaktorinhalt (18,07 g) wurde in eine PFA-Flasche gegeben und 5 min. mit Stickstoff gespült (18,02 g), aus dieser Lösung wurden 1,54 g entnommen und auf 1 1 mit dest. Wasser aufgefüllt und naßchemisch analysiert (Probe 2).

Aus den Analysendaten ergibt sich nun eine Katalysatorzusammensetzung von: **NEt**_{**3**} **x 5,75 HF x 0,08 HCl**

### Beispiel 6:

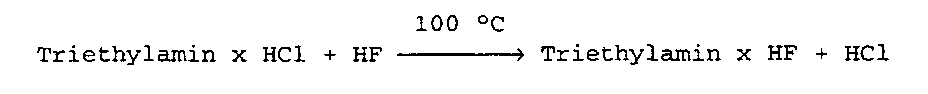

### Ansatz:

| **Stoff** | **Molmasse** | **Masse in g** | **Mol** |
|---|---|---|---|
| Tributylamin x HCl | 137,65 | 10,44 | 0,08 |
| HF | 20,01 | 21,3 | 1,06 |

### Durchführung:

In einem Laborautoklav wurde Triethylamin Hydrochlorid vorgelegt und verschlossen. Danach wurde HF zu gegeben und 30 min. bei ca. 100 °C Reaktortemperatur gekocht (Autoklav wurde 15 min. vorgeheizt). Nach dieser Zeit wurde der Autoklav aus dem Ölbad genommen und die Gasphase innerhalb von 15 min. abgelassen und analysiert (Probe 1). Der Reaktorinhalt (20,96 g) wurde in eine PFA - Flasche gegeben und 5 min. mit Stickstoff gespült(20,64 g), aus dieser Lösung wurden 1,1 g entnommen und auf 1 1 mit dest. Wasser aufgefüllt und ionenchromatografisch analysiert (Probe 2).

Aus den Analysendaten ergibt sich eine Katalysatorzusammensetzung von : **Triethylamin x 8,48 HF x 0,14 HCl.**

## Patentansprüche

1. Verfahren zur Herstellung von Fluor enthaltenden Verbindungen aus Chlor oder Brom enthaltenden Verbindungen durch Chlor-Fluor-Austausch oder Brom-Fluor-Austausch unter Verwendung von Oniumfluorid-HF-Addukten als Reagenz oder Katalysator, **dadurch gekennzeichnet, daß** man verbrauchtes Oniumfluorid-HF-Addukt kontinuierlich oder batchweise mittels HF in Anwesenheit einer flüssigen Carbonsäure regeneriert und/oder daß man verbrauchte Trialkylammoniumfluorid-HF-Addukte bzw. verbrauchte HF-Addukte cyclischer, gesättigter Amine mittels HF regeneriert.

2. Verfahren nach Anspruch 1, **dadurch gekennzeichnet, daß** das verbrauchte Oniumfluorid-HF-Addukt HCl oder HBr enthält, welche ausgetrieben werden.

3. verfahren nach Anspruch 2, **dadurch gekennzeichnet, daß** man freigesetztes HCl oder HBr mittels Durchleiten oder Überleiten von Inertgas abbläst.

4. Verfahren nach Anspruch 1, **dadurch gekennzeichnet, daß** man bei einer Temperatur von -20 bis 200 °C regeneriert.

5. Verfahren nach Anspruch 1, **dadurch gekennzeichnet, daß** man das Verhältnis von Amin zu HF bei der Regenerierung auf einen Wert zwischen 1:1,1 und 1:3,5 einstellt.

6. Verfahren nach Anspruch 1, **dadurch gekennzeichnet, daß** das verbrauchte Oniumfluorid-HF-Addukt aus der Herstellung von Säurefluoriden aus Säurechloriden oder aus der Herstellung von Fluoralkanen oder Fluorethern aus Chloralkanen oder Chlorethern stammt.

7. Verfahren nach Anspruch 1, **dadurch gekennzeichnet, daß** die Regenerierung gleichzeitig mit einer Fluorierungsreaktion unter Verwendung von HF als Fluorierungsreagenz durchgeführt wird.

8. Verfahren nach Anspruch 1, **dadurch gekennzeichnet, daß** man in Anwesenheit einer halogenierten Carbonsäure arbeitet.

9. Verfahren nach Anspruch 8, **dadurch gekennzeichnet, daß** man in Anwesenheit von Trifluoressigsäure regeneriert.

## Claims

1. A process for the preparation of fluorine-containing compounds from chlorine- or bromine-containing compounds by chlorine-fluorine exchange or bromine-fluorine exchange using onium fluoride-HF adducts as reagent or catalyst, **characterised in that** spent onium fluoride-HF adduct is regenerated continuously or batchwise by means of HF in the presence of a liquid carboxylic acid and/or that spent trialkylammonium fluoride-HF adducts or spent HF adducts of cyclic, saturated amines are regenerated by means of HF.

2. A process according to Claim 1, **characterised in that** the spent onium fluoride-HF adduct contains HCl or HBr, which is expelled.

3. A process according to Claim 2, **characterised in that** released HCl or HBr is blown off by means of passing inert gas over or through.

4. A process according to Claim 1, **characterised in that** regeneration is effected at a temperature from -20 to 200°C.

5. A process according to Claim 1, **characterised in that** the ratio of amine to HF during the regeneration is set to a value between 1:1.1 and 1:3.5.

6. A process according to Claim 1, **characterised in that** the spent onium fluoride-HF adduct comes from the preparation of acid fluorides from acid chlorides or from the preparation of fluoroalkanes or fluoroethers from chloroalkanes or chloroethers.

7. A process according to Claim 1, **characterised in that** the regeneration is performed simultaneously with a fluorination reaction using HF as fluorination reagent.

8. A process according to Claim 1, **characterised in that** operation is in the presence of a halogenated carboxylic acid.

9. A process according to Claim 8, **characterised in that** the regeneration is carried out in the presence of trifluoroacetic acid.

## Revendications

1. Procédé de préparation de composés contenant du fluor à partir de composés contenant du chlore ou du brome, par échange chlore-fluor ou échange brome-fluor en utilisant des adduits fluorure d'onium-HF comme réactif ou catalyseur, **caractérisé en ce que** l'on régénère l'adduit fluorure d'onium-HF utilisé, en continu ou de manière discontinue, à l'aide de HF en présence d'un acide carboxylique liquide et/ou **en ce que** l'on régénère à l'aide de HF l'adduit fluorure de trialkylammonium-HF utilisé ou l'adduit utilisé HF-amine cyclique saturée.

2. Procédé selon la revendication 1, **caractérisé en ce que** l'adduit fluorure d'onium-HF utilisé contient de l'HCl ou de l'HBr, qui sont chassés.

3. Procédé selon la revendication 2, **caractérisé en ce que** le HCl ou le HBr libéré est détendu par passage ou circulation d'un gaz inerte.

4. Procédé selon la revendication 1, **caractérisé en ce que** l'on régénère à une température allant de -20 à 200 °C.

5. Procédé selon la revendication 1, **caractérisé en ce que** l'on ajuste le rapport de l'amine au HF pendant la régénération à une valeur comprise entre 1:1,1 à 1:3,5.

6. Procédé selon la revendication 1, **caractérisé en ce que** l'adduit fluorure d'onium-HF utilisé provient de la préparation de fluorures d'acide à partir de chlorures d'acide ou de la préparation de fluoroalcanes ou de fluoroéthers à partir de chloroalcanes ou de chloroéthers.

7. Procédé selon la revendication 1, **caractérisé en ce que** l'on réalise la régénération simultanément à une réaction de fluoration en utilisant le HF comme réactif de fluoration.

8. Procédé selon la revendication 1, **caractérisé en ce que** l'on travaille en présence d'un acide carboxylique halogéné.

9. Procédé selon la revendication 8, **caractérisé en ce que** l'on régénère en présence d'acide trifluoroacétique.
